# EUROPEAN PATENT APPLICATION

(11) **EP 4 105 340 A1**
(43) Date of publication of application: **21.12.2022**
(21) Application number: 21753086.4
(22) Date of filing: 04.02.2021
(51) Int. Cl.: C12Q 1/6874, C12M 1/34, G01N 33/53

(54) **ANALYSIS METHOD, ANALYSIS SYSTEM, AND SURFACE FOR ANALYSIS**

(30) Priority: 14.02.2020 JP 2020023365
(71) Applicant: Sony Group Corporation, Minato-Ku, Tokyo, 108-0075 (JP)
(72) Inventor: MATSUMOTO Masahiro, Tokyo 108-0075 (JP); NAKAGAWA Kazuhiro, Tokyo 108-0075 (JP)
(74) Representative: D Young & Co LLP
(86) International application number: PCT/JP2021/004130
(87) International publication number: WO 2021/161893

(57) **Abstract**

An object is to provide a method capable of associating position information of a cell with a cell constituent at a single-cell resolution.

That is, the present technology provides an analysis method including: an imaging step of imaging a specimen in a state in which the specimen is being overlapped with a surface to which a molecule having a linker cleavable by stimulation, a barcode sequence, and a target capture portion is immobilized via the linker; an association step of associating a position of a cell with the barcode sequence of the molecule at the position by using a specimen image obtained by the imaging; a cleavage step of selectively stimulating the position of the cell to cleave the linker of the molecule at the position; and a binding step of binding the molecule released from the surface by the cleavage with a constituent of the cell via the target capture portion of the molecule.

## Description

### TECHNICAL FIELD

The present technology relates to an analysis method, an analysis system, and an analysis surface. More specifically, the present technology relates to an analysis method, an analysis system, and an analysis surface for acquiring the type and amount of constituent contained in a cell in association with position information of the cell.

### BACKGROUND ART

In order to analyze a biological tissue, the type and amount of mRNA contained in cells forming the biological tissue are measured. In the analysis of the biological tissue, not only the type and amount of mRNA contained in the cells but also position information of the cells is important. In view of this, there have been proposed so far some methods for acquiring position information of cells contained in a biological tissue and information regarding mRNA contained in the cells in association with each other.

For example, Non-Patent Document 1 cited below discloses a method called spatial transcriptomics. A probe used in this method contains a cleavage site, a T7 amplification and sequencing handle, a spatial barcode, a UMI, and an mRNA capture region (Fig. 2 in Non-Patent Document 1). In the method, a tissue section is placed on a glass slide to which the probe is immobilized, an mRNA in the tissue section is captured by the molecule, and is then subjected to reverse transcription to synthesize a cDNA. The synthesized cDNA is cleaved at the cleavage site, is collected in a tube, and is subjected to an analysis step such as a sequence.

### CITATION LIST

### NON-PATENT DOCUMENT

Non-Patent Document 1: Patrik L. Stahl et al., Visualization and analysis of gene expression in tissue sections by spatial transcriptomics, Science, 1 July 2016, Vol. 353, Issue 6294, pp. 78-82

### SUMMARY OF THE INVENTION

### PROBLEMS TO BE SOLVED BY THE INVENTION

It is considered that, if position information of a cell and information regarding a constituent contained in the cell can be associated with each other for each cell forming the biological tissue, it is possible to analyze the biological tissue in more detail. In view of this, an object of the present technology is to provide a method capable of associating position information of a cell with a cell constituent at a single-cell resolution.

### SOLUTIONS TO PROBLEMS

The inventors of the present technology have found that the above problem can be solved by a specific analysis method.

That is, the present technology provides an analysis method including:
an imaging step of imaging a specimen in a state in which the specimen is being overlapped with a surface to which a molecule having a linker cleavable by stimulation, a barcode sequence, and a target capture portion is immobilized via the linker;
an association step of associating a position of a cell with the barcode sequence of the molecule at the position by using a specimen image obtained by the imaging;
a cleavage step of selectively stimulating the position of the cell to cleave the linker of the molecule at the position; and
a binding step of binding the molecule released from the surface by the cleavage with a constituent of the cell via the target capture portion of the molecule.

The specimen may include a tissue sample.

In the cleavage step, the position of the cell may be selectively stimulated so as not to cleave a linker of a molecule at a position other than the position of the cell.

The stimulation may be light stimulation.

The binding step may include a moving step of moving the molecule toward the cell by applying an electric field, a magnetic field, or a centrifugal force.

The binding step may include a moving step of moving the molecule toward the cell by natural diffusion.

The binding step may include an incubation step of binding the molecule with the constituent of the cell.

The analysis method of the present technology may further include an analysis step of analyzing a conjugate of the molecule and the constituent of the cell after the binding step.

The conjugate may be subjected to a sequencing process in the analysis step.

The analysis step may include a two-dimensional mapping step of performing two-dimensional mapping on the basis of a result of the association in the association step by using a result of the analysis and the specimen image.

Further, the present technology also provides an analysis system including:
an analysis substrate having a surface to which a molecule having a linker cleavable by stimulation, a barcode sequence, and a target capture portion is immobilized via the linker;
an imaging device that images a specimen overlapped with the analysis substrate;
an association unit that associates a position of a cell selected in a specimen image obtained by the imaging with the barcode sequence of the molecule at the position; and
a stimulation provision device that selectively stimulates the position of the cell, in which
a conjugate in which the molecule released from the surface due to the stimulation binds with a constituent of the cell via the target capture portion of the molecule is used as an analysis target.

The specimen may include a tissue sample.

The stimulation provision device may be formed to selectively stimulate the position of the cell, without cleaving a linker of a molecule at a position other than the position of the cell.

The stimulation provision device may be a light irradiation device.

The analysis system may include an electric field application device, a magnetic field application device, or a centrifugal force application device that applies an electric field, a magnetic field, or a centrifugal force for moving, toward the cell, the molecule released from the surface due to the stimulation by the stimulation provision device.

The analysis system may further include an incubation device that prompts the molecule released from the surface due to the stimulation by the stimulation provision device to bind with the constituent of the cell.

The analysis system may further include an analysis device that analyzes the conjugate of the molecule released from the surface due to the stimulation by the stimulation provision device and the constituent of the cell.

The analysis device may be a sequencer.

The analysis system may further include a two-dimensional mapping unit that performs two-dimensional mapping on the basis of a result of the association by the association unit by using a result of the analysis by the analysis device and the specimen image obtained by the imaging.

Further, the present technology also provides an analysis surface, in which
a molecule having a cleavable linker, a barcode sequence, and a target capture portion is immobilized to the analysis surface via the linker, and
the barcode sequence is used to provide information regarding a position where the molecule having the barcode sequence is immobilized.

### BRIEF DESCRIPTION OF DRAWINGS

Fig. 1 shows an example of a flowchart of an analysis method according to the present technology.
Fig. 2 is a schematic view for describing an operation in each step included in the analysis method according to the present technology.
Fig. 3 is a schematic view showing an analysis surface used in the analysis method according to the present technology and a molecule immobilized to the surface.
Fig. 4 is a schematic view for describing an association step included in the method according to the present technology.
Fig. 5 is a block diagram of an example of an analysis system according to the present technology.

### MODE FOR CARRYING OUT THE INVENTION

Hereinafter, preferred modes for carrying out the present technology will be described. Note that embodiments described below are typical embodiments of the present technology, and the scope of the present technology is not limited to these embodiments. Note that the present technology will be described in the following order.
1. First embodiment (analysis method)
   (1) Description of first embodiment
   (2) Example of first embodiment
      (2-1) Imaging target preparation step
      (2-2) Imaging step
      (2-3) Association step
      (2-4) Cleavage step
      (2-5) Binding step
      (2-6) Analysis step
2. Second embodiment (analysis system)
3. Third embodiment (analysis surface)

### 1. First embodiment (analysis method)

### (1) Description of first embodiment

In an analysis method of the present technology, a specimen is imaged in a state in which the specimen is being overlapped with a surface to which a molecule having a linker cleavable by stimulation, a barcode sequence, and a target capture portion is immobilized via the linker. Then, a position of a cell contained in the specimen is associated with the barcode sequence of the molecule at the position by using a specimen image obtained by the imaging. After the association is performed, the position of the cell to be analyzed is stimulated, and the linker of the molecule present at the position is cleaved. The molecule is released from the surface due to the cleavage, and the molecule binds with a constituent of the cell to be analyzed via the target capture portion.

The molecule thus bound with the constituent of the cell has the barcode sequence, and the barcode sequence is associated with the position of the cell. Therefore, when the molecule bound with the constituent of the cell is analyzed (for example, identification of the constituent of the cell, identification of the barcode sequence, and the like), it is possible to acquire information regarding the constituent of the cell in association with position information of the cell. Further, in the analysis method, the specimen image is also acquired as described above. Therefore, for example, it is possible to map data regarding the constituent of the cell on the specimen image.

In the present technology, the specimen may be an immobilized specimen, for example, a frozen section or an FFPE section. Analysis methods of related arts are applicable only to the frozen section or the FFPE section in many cases. However, the analysis method of the present technology is applicable to both the sections. The specimen may be, for example, a tissue sample and may particularly be a biological tissue sample.

In the analysis method of the present technology, the position of the cell is selectively stimulated, and the molecule present at the position is released from the surface and is then bound with the constituent of the cell. More preferably, in the cleavage step, the position of the cell is selectively stimulated so as not to cleave a linker of a molecule at a position other than the position of the cell. This makes it possible to analyze the constituent of the cell (for example, mRNA or protein) at the single-cell resolution. That is, the analysis method of the present technology may be an analysis method of analyzing a constituent of a cell contained in a specimen at the single-cell resolution.

Further, it is possible to comprehensively analyze constituents of various cells contained in the specimen by using barcode sequences associated with position information of the cells. For example, in analysis of mRNA in a tissue sample according to related arts, a specific region can be designated on the basis of morphological information or immunostaining information of cells in the tissue sample, be cut out with a laser or the like, and then be subjected to mRNA analysis. In the present technology, it is possible to comprehensively perform expression information of mRNA in a tissue sample for each cell contained in the tissue sample, without, for example, cutting out the tissue sample. That is, the analysis method of the present technology may be an analysis method of analyzing constituents of a plurality of cells contained in a specimen at the single-cell resolution.

### (2) Example of first embodiment

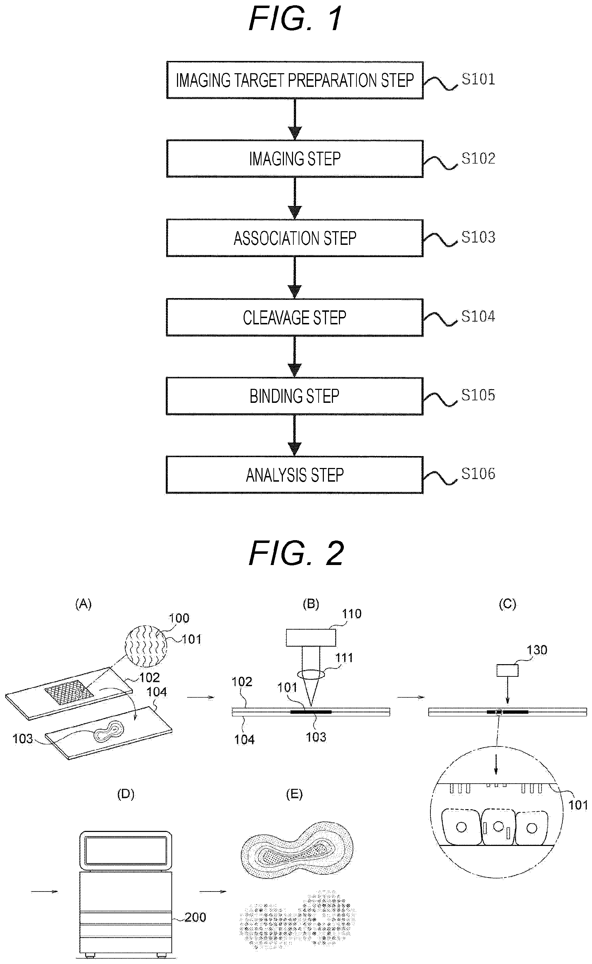

Fig. 1 shows an example of a flowchart of the analysis method according to the present technology. As shown in Fig. 1, the analysis method of the present technology can include an imaging target preparation step S101, an imaging step S102, an association step S103, a cleavage step S104, a binding step S105, and an analysis step S106. Each step will be described below.

### (2-1) Imaging target preparation step

In the imaging target preparation step S101, an imaging target in the imaging step S102 described later is prepared. The imaging target may be a laminate obtained by overlapping a specimen with a surface to which a molecule having a linker cleavable by stimulation, a barcode sequence, and a target capture portion is immobilized via the linker.

In the imaging target preparation step S101, as shown in, for example, (A) of Fig. 2, an analysis substrate (for example, a glass slide or the like) 102 having a surface 101 to which a plurality of molecules 100 is immobilized is overlapped with a substrate (for example, a glass slide or the like) 104 on which a specimen 103 is placed. The overlap may be performed so that the surface 101 and the specimen 103 face each other, and, for example, the surface 101 and the specimen 103 may be in contact with each other via a buffer or the like. Further, after the surface 101 and the specimen 103 are overlapped, the laminate of the analysis substrate 102 and the substrate 104 may be immersed in a buffer such as PBS.

A positional relationship between the substrates 102 and 104 can be fixed so that a positional relationship between the surface 101 and the specimen 103 thus overlapped is not changed in steps described later (in particular, the imaging step S102 to the binding step S105).

Each of the molecules 100 has a linker cleavable by stimulation, a barcode sequence, and a target capture portion.

In this specification, the molecule (that is, the molecule having the linker, the barcode sequence, and the target capture) is used to capture a target and can also be referred to as a target capture molecule. The target capture molecule is a name indicating a molecule for use in the present technology and can be used in this specification to, for example, refer to the molecule that has captured the target and also to refer to the molecule in which the linker has been cleaved in the cleavage step described later. The target capture molecule may be, for example, either a single molecule or a complex molecule. The single molecule may mean, for example, one type of molecule having a plurality of functions and may be, for example, one nucleic acid (for example, DNA or RNA) having a nucleic acid portion configured as the linker, a nucleic acid portion configured as the barcode sequence, and a nucleic acid portion configured as the target capture portion. The complex molecule may be, for example, a molecular assembly containing two or more types of molecules (for example, a bound substance of two or more types of molecules) and may be, for example, a conjugate of a nucleic acid having a nucleic acid portion configured as the linker and a nucleic acid portion configured as the barcode sequence and a polypeptide (for example, a protein or a part thereof, an oligopeptide, or the like) configured as the target capture portion.

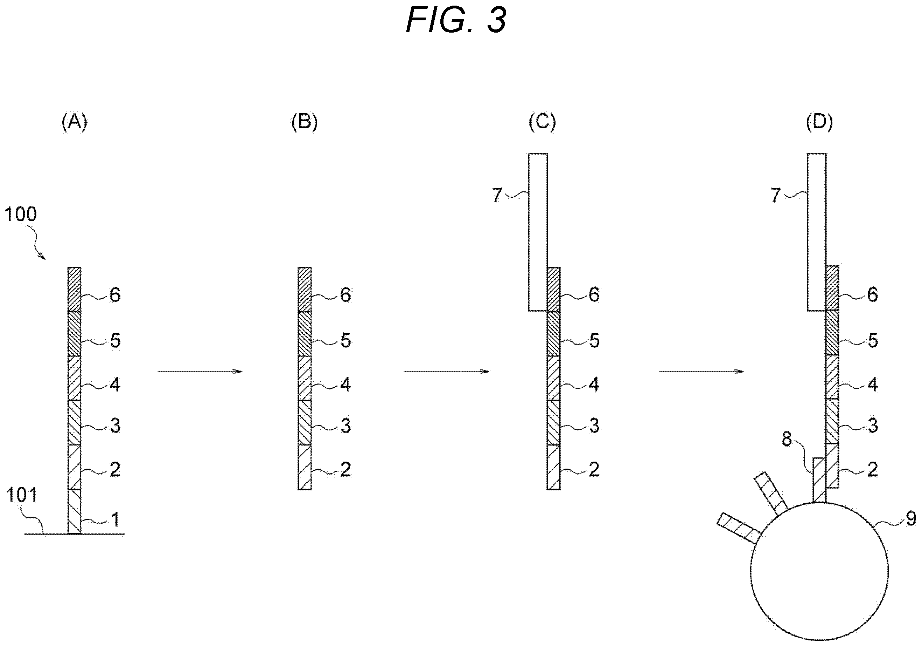

An example of a structure of the molecule 100 will be described with reference to Fig. 3. The molecule 100 in Fig. 3 has a linker 1, a collection sequence portion 2, an amplification sequence portion 3, a barcode sequence portion 4, a unique molecular identifier (UMI) portion 5, and a target capture portion 6. Further, the molecule 100 is immobilized to the surface 101 via the linker 1.

The collection sequence portion 2, the amplification sequence portion 3, the barcode sequence portion 4, and the UMI portion 5 may be configured as a continuous nucleic acid (in particular, DNA). In a case where the target capture portion 6 is a nucleic acid, not only the collection sequence portion 2, the amplification sequence portion 3, the barcode sequence portion 4, and the UMI portion 5, but also the target capture portion 6 may be configured as a continuous nucleic acid (in particular, DNA). In those cases, for example, an end near a portion where the molecule 100 is immobilized to the surface 101 can be the 5'-end, and the other end can be the 3'-end.

Components of the molecule 100 will be described below.

The linker 1 may be cleavable by stimulation, is cleavable by, for example, light stimulation or temperature stimulation, and is preferably cleavable by light stimulation. Light stimulation is particularly suitable for selectively stimulating a specific position in the cleavage step described below.

The linker 1 can contain, as, for example, the linker cleavable by light stimulation, any one selected from an arylcarbonylmethyl group, a nitroaryl group, a coumarin-4 ylmethyl group, an arylmethyl group, a metal-containing group, and other groups. Those groups may be groups described in, for example, Photoremovable Protecting Groups in Chemistry and Biology: Reaction Mechanisms and Efficacy, Chem. Rev. 2013, 113, 119-191.

For example, the arylcarbonylmethyl group may be a phenacyl group, an o-alkylphenacyl group, or a p-hydroxyphenacyl group. The nitroaryl group may be, for example, an o-nitrobenzyl group, an o-nitro-2 phenethyloxycarbonyl group, or o-nitroanilide. The arylmethyl group may be, for example, one into which a hydroxy group is introduced or one into which no hydroxy group is introduced.

In a case where the linker 1 is cleavable by light stimulation, the linker may be preferably cleaved by light having a wavelength of 360 nm or more. The linker may be preferably cut at an energy of 0.5 µJ/µm² or less. (Light-sheet fluorescence microscopy for quantitative biology, Nat Methods. 2015 Jan;12(1):23-6. doi:10.1038/nmeth.3219.). By adopting the linker cut by light having the above wavelength or at the above energy, it is possible to reduce cell damage (in particular, cut of DNA or RNA or the like) that may occur when light stimulation is applied.

In a case where the linker 1 is cleavable by temperature stimulation, the linker 1 can contain, for example, a temperature-responsive polymer. The temperature-responsive polymer can change from hydrophilic to hydrophobic or from hydrophobic to hydrophilic in response to, for example, a temperature change. By such a change, the target capture molecule can be released from the surface 1.

It is particularly preferable that the linker may be cleaved by light in a short wavelength region, specifically, light in a wavelength region of 360 nm to 410 nm or may be cleaved by light in a near infrared region or an infrared region, specifically, light in a wavelength region of 800 nm or more. In a case where the linker is efficiently cut by light having a wavelength in a visible light region, it may be difficult to handle the analysis surface. Therefore, the linker is preferably cleaved by light in the short wavelength region or light in the near infrared region or the infrared region.

The collection sequence portion 2 contains a nucleic acid used to collect the molecule 100 released from the surface 101 in the analysis step described later. The nucleic acid may be DNA or RNA and is particularly DNA. For example, as shown in (D) of Fig. 3, a sequence of the nucleic acid contained in the collection sequence portion 2 is complementary to a sequence of a nucleic acid 8 immobilized to a bead 9. The molecule 100 having the collection sequence portion 2 can be efficiently collected by the bead 9 to which the plurality of nucleic acids 8 is immobilized. A base sequence of the nucleic acid contained in the collection sequence portion 2 may be appropriately set by those skilled in the art.

The amplification sequence portion 3 can contain, for example, a nucleic acid having a primer sequence used to amplify the nucleic acid or a promoter sequence used to transcribe the nucleic acid in the analysis step described later. The nucleic acid may be DNA or RNA and is particularly DNA. The amplification sequence portion 3 may have both the primer sequence and the promoter sequence. The primer sequence may be, for example, a PCR handle. The promoter sequence may be, for example, a T7 promoter sequence.

The barcode sequence portion 4 contains a nucleic acid having a barcode sequence. The nucleic acid may be particularly DNA or RNA and is more particularly DNA. The barcode sequence is used to, for example, identify a position of the target capture molecule on the surface 101. The barcode sequence can be used as an identifier for distinguishing a target capture molecule having a certain barcode sequence from target capture molecules having other barcode sequences. The barcode sequence may be associated with information regarding a position where the target capture molecule having the barcode sequence is immobilized (hereinafter, also referred to as "position information"). The position information may be for identifying the position on the surface 101 and is, for example, information regarding XY coordinates, but is not limited thereto. An ID number may be allocated to the barcode sequence associated with the position information. The ID number can be used in steps in and after the imaging step. The ID number may correspond to the barcode sequence on a one-to-one basis and may be used as data corresponding to the barcode sequence in the steps in and after the imaging step.

A plurality of target capture molecules immobilized in a certain region of the surface 101 can have the same barcode sequence. Therefore, the certain region and the barcode sequence are associated with each other. By setting a size of the certain region to be smaller than a size of a cell, the target capture molecules having the barcode sequence can be associated with a position where one cell is present. As described above, the surface used in the analysis method of the present technology can have a plurality of regions to each of which a plurality of target capture molecules having the same barcode sequence is immobilized. The barcode sequence may be different in each region. A size of each region is preferably smaller than the size of the cell and can be, for example, 50 µm or less, preferably 10 µm or less, and more preferably 5 µm or less.

In one embodiment of the present technology, a target capture molecule having a barcode sequence whose sequence is known can be immobilized in a predetermined region. For example, the surface 101 has a plurality of regions, and a plurality of target capture molecules immobilized to each of the plurality of regions can have the same barcode sequence. The plurality of regions can be set to be smaller than the size of the cell to be analyzed. Regarding the surface 101 formed as described above, each of the plurality of regions can be associated with the barcode sequence of the plurality of target capture molecules immobilized to each region.

The region in which the target capture molecules having the same barcode sequence are immobilized as described above will also be referred to as a spot in this specification. A size of the spot can be, for example, 50 µm or less, preferably 10 µm or less, and more preferably 5 µm or less.

In the surface 101 formed as described above, a barcode sequence of certain target capture molecules and a position where the certain target capture molecules are present can be associated with each other when the target capture molecules are immobilized to the surface 101. For the immobilization, for example, biotin is bound with the linkers 1 of the target capture molecules, streptavidin is bound with the surface 101 to which the target capture molecules are to be immobilized, and the biotin binds with the streptavidin, whereby the target capture molecules are immobilized to the surface 101.

In another embodiment of the present technology, target capture molecules having barcode sequences may be randomly arranged on the surface 101. In this case, after the target capture molecules having the barcode sequences are immobilized to the surface 101, the barcode sequences of the immobilized target capture molecules are read, whereby a barcode sequence of certain target capture molecules is associated with a position where the certain target capture molecules are present. The reading can be performed by a method such as sequencing by synthesis, sequencing by ligation, or sequencing by hybridization, for example.

In this embodiment, for example, a bead (for example, gel bead) bound with a plurality of target capture molecules having the same barcode sequence may be used, and the bead (for example, gel bead) can be immobilized to the surface 101. A size of the bead (for example, gel bead) can be, for example, 50 µm or less, preferably 10 µm or less, and more preferably 5 µm or less. In order to bind the target capture molecules with the bead (for example, gel bead), for example, a combination of biotin and streptavidin may be used. For example, biotin is bound with the linkers 1 of the target capture molecules, streptavidin is bound with the bead, and the biotin binds with the streptavidin, whereby the target capture molecules are immobilized to the bead.

The surface 101 may have a plurality of recesses. One spot or one bead in the embodiment may be placed in each of the plurality of recesses. The spot or the bead can be more easily placed on the surface 101 because of the plurality of recesses. The recess preferably has a size in which one bead can be placed, for example. The recess may have a shape such as a circle, an ellipse, a hexagon, or a quadrangle, but is not limited thereto.

Further, in the surface 101, a surface state of a surface portion on which the spot or the bead is placed may be different from those of other surface portions. For example, the surface portion on which the spot or the bead is placed may be hydrophilic, and the other surface portions may be hydrophobic, or the other surface portions may be hydrophobic and have protrusions. Examples of a method of imparting hydrophilicity to the surface include reactive ion etching in the presence of oxygen and irradiation with deep ultraviolet light in the presence of ozone. In those methods, it is possible to use a mask whose portions to which hydrophilicity is to be imparted are penetrated. Further, examples of a method of imparting hydrophobicity to the surface include a silicone spray (spray-on-silicone), and, for example, Techspray 2101-12S or the like may be used. Also in a case of imparting hydrophobicity, for example, it is possible to use a mask whose portions to which hydrophobicity is to be imparted are penetrated.

For example, it is also possible to synthesize the target capture molecules on the substrate by using a DNA microarray preparation technology or the like. For example, it is possible to synthesize the target capture molecules at a specific position by using a technology such as a digital mircomirror device (DMD) used for photolithography, a liquid crystal shutter, or a spatial light phase modulator. A method for the synthesis is described in, for example, Basic Concepts of Microarrays and Potential Applications in Clinical Microbiology, CLINICAL MICROBIOLOGY REVIEWS, Oct. 2009, pp. 611-633. Note that, in a case where the target capture molecules are synthesized on the substrate by the synthesis, information regarding a position where the target capture molecules are synthesized is acquired when the target capture molecules are synthesized, and the barcode sequence is associated with the position information. At that time, an ID number may be given.

In one embodiment of the present technology, all the target capture molecules immobilized to the surface may have a common oligo sequence. By using a fluorescently labeled nucleic acid having a sequence complementary to the oligo sequence, it is possible to confirm a position where the target capture molecules are immobilized (in particular, a position of the spot or a position of the bead), and it is particularly possible to confirm the position in a dark field. Further, in a case where there is no recess or protrusion described above on the surface, it may be difficult to grasp the position where the target capture molecules are immobilized. In this case, the fluorescent label makes it easier to grasp the position where the target capture molecules are immobilized.

The UMI portion 5 may contain a nucleic acid, particularly DNA or RNA, and more particularly DNA. The UMI portion 5 can have a sequence of, for example, 5 bases to 30 bases, particularly 6 bases to 20 bases, and more particularly 7 bases to 15 bases.

The UMI portion 5 can be configured so that the target capture molecules immobilized to the surface 101 have different sequences. For example, in a case where the UMI portion has a nucleic acid sequence of 10 bases, the type of the UMI sequence is four to the tenth power, that is, one million or more.

The UMI portion 5 can be used to quantify a target molecule. For example, a UMI sequence is added to cDNA obtained by reverse transcription of mRNA. A large number of cDNAs obtained by amplifying a cDNA transcribed from one mRNA molecule have the same UMI sequence, but a large number of cDNAs obtained by amplifying a cDNA transcribed from another mRNA molecule having the same sequence as the mRNA have a different UMI sequence. Therefore, the number of copies of mRNA can be determined by counting the number of types of UMI sequences having the same cDNA sequence.

For example, the UMI portion 5 can be configured so that the plurality of target capture molecules having the same barcode sequence immobilized to the spot or the bead has different sequences. That is, the plurality of target capture molecules immobilized to the spot or the bead can have the same barcode sequence and have different UMIs.

The target capture portion 6 has a component for capturing a molecule contained in a cell. The component can be, for example, a nucleic acid or a protein. In a case where the component is a nucleic acid, the nucleic acid may be, for example, a poly-T sequence in order to comprehensively capture mRNAs contained in the cell. Alternatively, the nucleic acid can have a sequence complementary to a target sequence. In a case where the component is a protein, the protein may be, for example, an antibody. The component may be an aptamer or a molecular imprinted polymer.

The target capture portion 6 may contain two or more types of components for capturing the molecule contained in the cell. The target capture portion 6 may contain both the protein and the nucleic acid and may contain, for example, both the antibody and the poly-T sequence. Therefore, it is possible to simultaneously detect both protein and mRNA.

The surface 101 can preferably be a surface of a transparent substrate. The entire substrate may be transparent, or only a portion thereof to which the target capture molecules are to be immobilized may be transparent. The surface of the substrate is preferably planar in order to favorably bring the surface into contact with a specimen. The transparent substrate may be, for example, a glass substrate or a resin substrate. The substrate may be, for example, a glass slide. Because the substrate is transparent, it is possible to image a specimen in the imaging step described later. Further, because the substrate is transparent, association can be easily performed in the association step described later.

A specimen 4 can be, for example, a specimen containing a cell and more particularly an immobilized specimen containing a cell. The specimen may be a biological tissue specimen, particularly a frozen tissue specimen or a formalin-fixed paraffin-embedded (FFPE) specimen, and more particularly a frozen tissue section or an FFPE section. The specimen 4 may be mounted on, for example, a substrate and can particularly be mounted on a surface of a transparent substrate. The entire substrate may be transparent, or only a portion thereof on which the specimen is to be mounted may be transparent. The transparent substrate may be, for example, a glass substrate or a resin substrate. The substrate may be, for example, a glass slide. The surface of the substrate is preferably planar in order to favorably bring the surface into contact with the surface to which the target capture molecules are immobilized.

The specimen 4 may be stained in order to more easily perform segmentation of cells described later. The staining may be, for example, cell membrane staining, hematoxylin eosin (HE) staining, DAPI staining, or a combination of two or more thereof. For the cell membrane staining, a cell membrane staining reagent that is incorporated into a lipid bilayer membrane to emit fluorescence may be used, and the reagent may be appropriately selected by those skilled in the art. The HE staining can be used for bright-field observation, for example, to identify cell morphology. The DAPI staining may be used for nuclear staining.

### (2-2) Imaging step

In the imaging step S102, for example, as shown in (B) of Fig. 2, the specimen 103 is imaged in a state in which the surface 101 and the specimen 103 are being overlapped. The imaging can be performed at a resolution at which individual cells contained in the specimen 103 can be recognized. An imaging element 110 may be, for example, a CCD or CMOS.

The imaging may be performed by, for example, the imaging element 110 via an objective lens 111. That is, the captured image can be a microscopic image. A magnification of the objective lens 111 may be appropriately selected according to the size of the cells.

The imaging may be bright-field imaging or dark-field imaging, or both bright-field imaging and dark-field imaging may be performed. The imaging may be performed once or a plurality of times. For example, the imaging may be performed once or a plurality of times for a partial region selected by a user or a control unit or may be performed once or a plurality of times so as to comprehensively cover the entire or a part of the specimen 103.

The imaging by the imaging element 110 can be controlled by the control unit (not shown) connected to the imaging element 110. The control unit may include, for example, a hard disk, a CPU, and a memory, and a function of the control unit can be achieved by, for example, a general-purpose computer or information processing device.

Further, the control unit may be provided in the imaging element 110. The imaging element including the control unit may be formed as, for example, a one-chip semiconductor device having a laminated structure in which a plurality of dies (for example, two or three dies) is laminated. One of the dies includes a plurality of pixels arrayed side by side in two dimensions. Components (for example, the CPU, the memory, and the like) for achieving the functions of the control unit can be mounted on the remaining dies. An example of the imaging element including the control unit is, for example, an imaging element disclosed in WO 2018/051809. By using the imaging element including the control unit as the imaging element, it is possible to perform various types of processing, without outputting specimen image data to the outside of the imaging element. This leads to an increase in speed of information processing.

The imaging element 110 can transmit the specimen image data obtained by the imaging to the control unit. The control unit receives the specimen image data and uses the specimen image data in the subsequent steps.

Further, the specimen image data received by the control unit may be stored in, for example, a storage unit connected to the control unit. The storage unit may be a general-purpose storage device. The control unit can acquire the specimen image data from the storage unit in a case of performing the subsequent steps.

### (2-3) Association step

In the association step S103, the position of the cell and the barcode sequence of the target capture molecules at the position are associated with each other by using the specimen image obtained by the imaging in the imaging step S102. The association may be performed via the position information associated with the barcode sequence in advance. In a case where the ID number is allocated to each barcode sequence, the position of the cell and the ID number may be associated. Therefore, the position of the cell and the barcode sequence may be associated via the ID number.

The position of the cell can mean a region occupied by the cell in the specimen image. In order to identify the region, image processing may be performed on the specimen image, for example, cell segmentation can be performed thereon. The cell segmentation makes it easier to identify the target capture molecules present at the position of the cell.

For example, in the association step S103, position information of the cell to be analyzed among the cells in the specimen image is acquired. The position information of the cell may be acquired by using image data obtained by the segmentation. Because the specimen image is obtained by imaging the surface 101 and the specimen 103 thus overlapped, the position information of the cell corresponds to position information of the target capture molecules at the position of the cell. Here, as described above, the position information of the target capture molecules is associated with the barcode sequence of the target capture molecules in the imaging target preparation step S101. Therefore, by acquiring the position information of the cell in the specimen image, it is possible to associate the position of the cell with the barcode sequence of the target capture molecules at the position.

The association can be preferably performed so that one barcode sequence is not associated with positions of two or more cells. For example, two or more cells are present in one spot described above in some cases. In this case, a barcode sequence of target capture molecules immobilized to the one spot is preferably not associated with any position of the two or more cells.

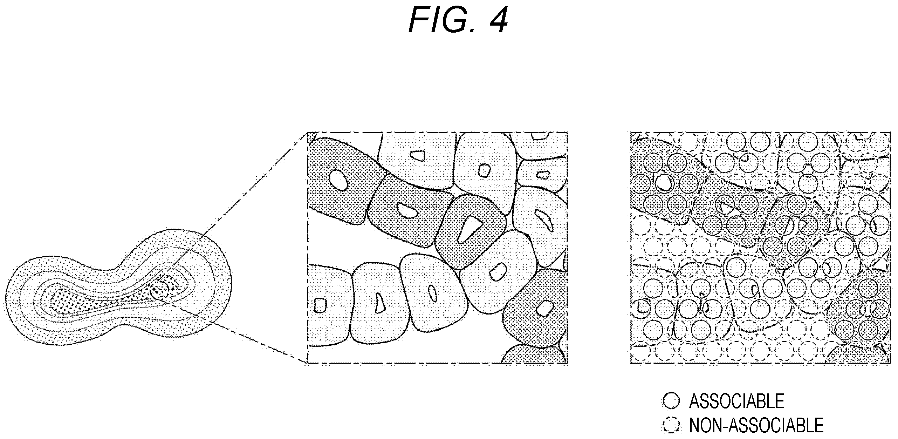

An example of how to distinguish between a spot to be associated and a spot not to be associated among spots on the surface will be described with reference to Fig. 4.

When a part of a specimen image of a tissue in a left part of Fig. 4 is enlarged or an enlarged image of the part thereof is acquired, it is possible to visually recognize cells in a tissue sample as shown in a central part of Fig. 4. A specimen is imaged while being overlapped with an analysis surface and is thus imaged in a state in which the specimen is overlapped with a large number of circle spots as shown in a right part of Fig. 4, for example. Each circle spot has a plurality of target capture molecules having the same barcode sequence.

In the right part of Fig. 4, a large number of circle spots are shown for better understanding. However, those spots may not be or may be confirmed in the image acquired by the imaging element.

Note that, in a case where positions of the spots cannot be confirmed in the specimen image, the positions of the spots may be displayed in the specimen image by image processing.

In the specimen image in the central part of Fig. 4, it is possible to confirm that two types of cells are present. One type of cells is colored with dark gray, and the other type of cells is colored with light gray. In a case where, for those two types of cells, a position of each cell is associated with a barcode sequence of target capture molecules present at the position, spots indicated by solid-line circles are associated as shown in the right part of Fig. 4, for example. Each solid-line circle is present in a region of an image of one cell. As described above, the spots present in the region of the image of the one cell can be a target to be associated.

Meanwhile, for example, each dotted-line circle overlaps two or more cells, extends over a cell and a region outside the cell, or is present outside the region of the image of the cell. Those dotted-line circles may not be a target to be associated.

The association step S103 can be performed by, for example, the control unit. For example, the control unit can associate positions of all or some types of cells among the cells present in the specimen image with barcode sequences of molecules present at the positions of the cells. In a case where some types of cells are associated, the control unit can identify cells to be associated on the basis of, for example, characteristics of the cells (a shape, a size, a color, a pattern, or a combination thereof). For example, only a specific type of cells may be associated. For example, in the image in the central part of Fig. 4, positions of the cells with dark gray are associated with barcode sequences of target capture molecules present at the positions, whereas the cells with light gray are not associated.

Further, in a case where the imaging element including the control unit is used as the imaging element, it is possible to perform the association step S103, without outputting the specimen image data to the outside of the imaging element. Therefore, it is possible to perform the association step S103 at a higher speed.

The association step S103 may be performed in response to a user operation. For example, the user selects a cell to be associated from among the cells present in the specimen image by, for example, clicking a mouse. Then, the control unit can associate a position of the cell selected by the user with a barcode sequence of target capture molecules at the position.

### (2-4) Cleavage step

In the cleavage step S104, the position of the cell is selectively stimulated to cleave the linkers of the molecules at the position. For example, as shown in (C) of Fig. 2, the position of the selected cell can be irradiated with light by a stimulation provision device 130.

Positions where stimulation is provided may be positions of some of the cells associated in the association step S103 or may be positions of all the cells associated therein. It is preferable to selectively stimulate the position of the cell so as not to cleave linkers of target capture molecules at a position other than the position of the cell. The stimulation provision device 130 is preferably configured to selectively provide stimulation as described above. That is, in the cleavage step S104, linkers of target capture molecules having a barcode sequence that has not been associated may not be cleaved.

For example, in the image in the right part of Fig. 4, all the associated spots indicated by the solid-line circles may be stimulated. As described above, all the positions of the associated cells may be stimulated.

Alternatively, in the image in the right part of Fig. 4, positions indicated by the solid-line circle spots in the cells with dark gray or positions indicated by the solid-line circle spots in the cells with light gray may be stimulated. As described above, positions of some of the associated cells (in particular, cells of a certain type) may be stimulated.

Alternatively, in the image in the right part of Fig. 4, positions indicated by solid-line circle spots in one or a plurality of cells selected from among the associated cells may be stimulated. As described above, positions of some of the associated cells (in particular, selected cells) may be stimulated.

By the cleavage of the linkers in step S104, the target capture molecules are released from the surface 101. For example, as shown in (B) of Fig. 3, the linker 1 of the molecule 100 is cleaved, and the molecule 100 is released from the surface 101.

The stimulation may be, for example, light stimulation or temperature stimulation (also referred to as thermal stimulation) and may preferably be light stimulation. Light stimulation is particularly suitable for selectively stimulating a specific narrow range.

The cleavage step S104 may be performed by, for example, the control unit. More specifically, the control unit can drive the stimulation provision device to selectively stimulate the position of the cell. An example of an adoptable stimulation provision device will be described below.

In order to selectively apply light stimulation to the position of the cell, it is possible to use a light irradiation device as the stimulation provision device 130. The light irradiation device may be, for example, a digital micromirror device (DMD) or a liquid crystal display device. A micromirror included in the DMD can irradiate a selected position on the surface 101 with light. The liquid crystal display device may be, for example, a reflective liquid crystal display, and a specific example thereof includes SXRD (Sony Corporation). By controlling liquid crystal of the liquid crystal display device, it is possible to irradiate the selected position on the surface 101 with light.

Further, a liquid crystal shutter or a spatial light modulator may be used to selectively apply light stimulation to the position of the cell. Also by those units, light stimulation can be applied to the selected position.

A wavelength of the irradiation light may be appropriately selected by those skilled in the art according to the type of linkers contained in the target capture molecules.

For example, a combination of infrared light and an infrared light absorption material can be used to selectively apply temperature stimulation to the position of the cell. In this case, the stimulation provision device can be, for example, an infrared laser light generation device. For example, the substrate 102 having the surface 101 is made from an infrared light absorption material, and then the position of the cell is selectively irradiated with infrared light by the infrared laser light generation device, whereby temperature stimulation can be selectively applied to the position.

### (2-5) Binding step

In the binding step S105, the target capture molecules released from the surface 101 by the cleavage in step S104 are bound with constituents of the cell via the target capture portions of the target capture molecules.

For example, as shown in a schematic enlarged view surrounded by a one-dot chain line in (C) of Fig. 2, only linkers of target capture molecules at the position irradiated with light are cleaved and taken into a cell immediately therebelow. Then, for example, as shown in (C) of Fig. 3, a cell constituent 7 can bind with the target capture portion 6 of the target capture molecule 100 in the cell.

The binding step S105 can include a moving step of moving the target capture molecules toward the cell by applying an electric field, a magnetic field, or a centrifugal force. For example, because the nucleic acids contained in the target capture molecules have a negative charge, it is possible to move the target capture molecules toward the cell by a positive charge. For example, an electric field can be applied by placing a laminate of the substrate 102 and the substrate 104 between facing electrodes. Further, a metal thin film or transparent electrode such as an indium tin oxide (ITO) electrode having a thickness that does not hinder bright-field observation or dark-field observation (in particular, fluorescence observation) may be placed on the substrate 102 and the substrate 104. An electric field application device that applies an electric field, a magnetic field application device that applies a magnetic field, or a centrifugal force application device that applies a centrifugal force may be devices known in the art. Examples of the centrifugal force application device include a swing rotor type centrifugal force application device. The application of an electric field, a magnetic field, or a centrifugal force by those devices may be controlled by, for example, the control unit.

The binding step S105 may include a moving step of moving the target capture molecules toward the cell by natural diffusion.

In the moving step, a combination of the application of an electric field, a magnetic field, or a centrifugal force and natural diffusion may be used. The moving step preferably includes the application of an electric field. This makes it possible to increase a probability that the released target capture molecules are taken into the cell.

The binding step S105 may include an incubation step for binding the target capture molecules with the constituents of the cell. A time and temperature of the incubation step may be selected according to the target capture portions and the cell constituents captured by the target capture portions. The incubation step can be performed in a state in which, for example, a space between the surface 101 and the specimen is filled with a buffer. For example, in a case where both the target capture portions and the cell constituents are nucleic acids, for example, the incubation can be performed in a thermostatic chamber at 30°C to 40°C, particularly 30°C to 37°C for, for example, 5 hours to 30 hours, particularly 16 hours to 24 hours. For example, in a case where the target capture portions are antibodies and the cell constituents are constituents captured by the antibodies, the incubation can be performed at, for example, 0°C to 30°C, particularly 4°C to a room temperature (for example, 25°C) for, for example, 5 hours to 30 hours, particularly 16 hours to 24 hours.

In the binding step S105, the target capture molecules may be taken into the cell or can bind with a surface of the cell. Then, the target capture molecules bind with the constituents of the cell via the target capture portions of the target capture molecules. Thereafter, the surface 101 and the specimen 103 are separated, and unbound target capture molecules are removed by washing. A washing method may be, for example, immersing the specimen 103 in a buffer.

### (2-6) Analysis step

In the analysis step S106, a conjugate generated by binding the target capture molecule with the cell constituent in the binding step S105 can be analyzed. As shown in (D) of Fig. 2, the analysis can be performed by using an analysis device 200. For example, in the analysis step S106, the conjugate may be subjected to a sequencing process, and the analysis device 200 may be a sequencer. The sequencing process can be performed in a case where, for example, the cell constituent is a nucleic acid, particularly DNA or RNA, and more particularly mRNA. The sequencing process may be performed by a sequencer or may be performed by a next-generation sequencer or a sequencer by a Sanger method. In order to comprehensively analyze cells forming a tissue at a higher speed, the sequencing process can be performed by a next-generation sequencer.

In order to perform the sequencing process in the analysis step, the analysis step can further include a step of preparing a nucleic acid (for example, cDNA or the like) to be subjected to the sequencing process and a step of purifying the nucleic acid. By the preparation step and the purification step, for example, a library for performing a next-generation sequencing process may be prepared.

In the preparation of the library, the collection sequence portion 2 can be used as shown in, for example, (D) of Fig. 3. The molecule 100 bound with the cell constituent 7 can be collected by using the bead 9 to which a nucleic acid having a sequence complementary to the nucleic acid sequence contained in the collection sequence portion 2 is immobilized.

In the preparation step, the laminate of the substrates 102 and 104 bound in the binding step S105 can be washed by using, for example, a buffer such as PBS to remove unbound target capture molecules. Then, next, the substrate 102 is removed from the substrate 104, and, for example, a cDNA synthesis step of synthesizing cDNA from mRNA and an amplification step of amplifying the synthesized cDNA can be performed on the cell that has taken the target capture molecules. Before the cDNA synthesis step and the amplification step, a lysis step of lysing the cell may be performed. When a conjugate of the target capture molecules and a target is collected after the lysis step, it is possible to more efficiently perform the cDNA synthesis step and the amplification step.

After the preparation step, the purification step of purifying the nucleic acid obtained in the preparation step may be performed. The purification step may include, for example, a process of decomposing constituents other than the nucleic acid by using an enzyme such as Proteinase K. Further, a nucleic acid collection process may be performed in the purification step. In the nucleic acid collection process, for example, a commercially available nucleic acid purification reagent may be used, and examples thereof include magnetic beads such as AMPure XP. Note that dsDNA in the cell can also be collected in the purification step, but dsDNA can be prevented from being subjected to sequencing in the sequencing process. For example, when the target capture molecules have an adaptor sequence for a sequencing process (in particular, for a next-generation sequencing process), only a nucleic acid having the adaptor sequence can be subjected to sequencing.

In the analysis step S106, the cell constituents can be analyzed for each cell on the basis of a result of the sequencing process. For example, in the analysis step S106, the sequences of the mRNAs contained in the cell and/or the number of copies of each mRNA can be determined for each cell. Further, in the analysis step S106, the type and/or number of antigens or the type and/or number of transcription factors can be determined for each cell. Such analysis of the cell constituents for each cell can be performed on the basis of a barcode sequence of sequences determined by the sequencing process. For example, sequences having the same barcode sequence are selected from a large number of sequences determined by the sequencing process. The sequences having the same barcode sequence are based on target capture molecules taken into one cell. Therefore, analyzing the cell constituents for each barcode sequence means analyzing the cell constituents for each cell.

The analysis step S106 can include a two-dimensional mapping step of performing two-dimensional mapping on the basis of a result of the association in the association step S103 by using a result of the analysis and the specimen image. For example, in the analysis step S106, the analysis result of the cell constituents of each cell can be mapped on the specimen image obtained in the imaging step on the basis of the position information associated with the barcode sequence. By the mapping, for example, as shown in (E) of Fig. 2, it is possible to obtain a specimen image (an upper part in (E) of Fig. 2) and a mapping image (a lower part in (E) of Fig. 2) showing a distribution of the types of cell constituents at respective positions in the specimen image. On the basis of the images obtained by the mapping, it is possible to grasp which cell at which position contains which molecule in what amount. That is, it is possible to combine the position information obtained by the imaging with quantitative information obtained by the sequence analysis, and thus it is possible to comprehensively analyze the molecules in the tissue sample while holding spatial information at the single-cell resolution.

### 2. Second embodiment (analysis system)

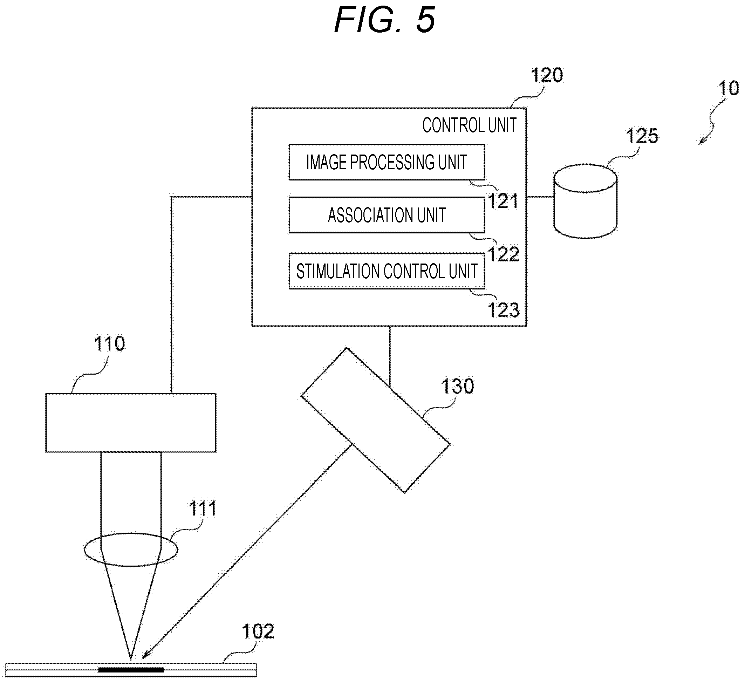

Fig. 5 is a block diagram of an example of an analysis system according to the present technology. As shown in Fig. 5, an analysis system 10 of the present technology includes the analysis substrate 102, the imaging element 110, a control unit 120, a storage unit 125, and the stimulation provision device 130.

The analysis substrate 102, the imaging element 110, and the stimulation provision device 130 may be those described in the above section 1, and the description thereof is also applied to the present embodiment.

The control unit 120 may be the control unit described in the above section 1, and the description thereof is also applied to the present embodiment. The control unit 120 can include, for example, an image processing unit 121, an association unit 122, and a stimulation control unit 123. Those units will be described in more detail below.

The image processing unit 121 processes a specimen image acquired by the imaging element 110. The image processing unit 121 can perform, for example, the cell segmentation described in the above section 1. Further, the image processing unit 121 can identify a cell to be associated in the association step from among cells present in the specimen image. The identification can be performed on the basis of, for example, characteristics of the cell (a shape, a size, a color, a pattern, or a combination thereof). The identification may also be performed on the basis of color data in the image. For example, it is possible to identify a cell emitting a certain fluorescence as the cell to be associated in the association step.

The association unit 122 associates a position of the cell with a barcode sequence of target capture molecules at the position of the cell. The association may be performed by using the image obtained by the segmentation.

For example, the association unit 122 can acquire position data of the cell on the basis of the specimen image, identify a barcode sequence to which position data corresponding to the position data is allocated, and associate the barcode sequence with the position of the cell.

The association unit 122 may perform the association of the position of the cell with the barcode sequence of the target capture molecules for all cells in the specimen image or some of the cells in the specimen image. For example, the association unit 122 can associate only some types of cells or only cells present in some regions among all the cells present in the specimen image.

The stimulation control unit 123 causes the stimulation provision device 130 to selectively stimulate the position of the cell associated by the association unit 122. The stimulation control unit 123 can drive the stimulation provision device 130 to stimulate all or some of the cells associated by the association unit 122. The stimulation provision device 130 is preferably configured to selectively stimulate the position of the cell, without cleaving linkers of target capture molecules at a position other than the position of the cell. The stimulation provision device 130 is preferably the light irradiation device described in the above section 1.

The control unit 120 may include, for example, a hard disk, a CPU, and a memory, and a function of the control unit can be achieved by, for example, a general-purpose computer or information processing device. Functions of the image processing unit 121, the association unit 122, and the stimulation control unit 123 can also be achieved by a general-purpose computer or information processing device.

As described in the above section (2-2), the control unit 120 may be provided in the imaging element 110. By using the imaging element including the control unit as the imaging element, it is possible to perform various types of processing, without outputting the specimen image data to the outside of the imaging element. This leads to an increase in speed of information processing. For example, the processing by the image processing unit 121, the association unit 122, and the stimulation control unit 123 described above can be performed at a higher speed.

In order to perform the binding step S105 described in the above section 1, the analysis system 10 can include, for example, an electric field or magnetic field application device that applies an electric field or magnetic field or a centrifugal force application device that applies a centrifugal force for moving, toward the cell, the target capture molecules released from the surface of the analysis substrate 102 due to the stimulation by the stimulation provision device 130. By the device, the target capture molecules are taken into the cell more efficiently.

In order to perform the binding step S105 described in the above section 1, the analysis system 10 can further include an incubation device for promoting a cell constituent to bind with the target capture molecule released from the surface of the analysis substrate 102 due to the stimulation by the stimulation provision device 130. The incubation device can include, for example, a thermostatic chamber.

In order to perform the analysis step S106 described in the above section 1, the analysis system 10 can further include an analysis device for analyzing the conjugate of the cell constituent with the target capture molecule released from the surface of the analysis substrate 102 due to the stimulation by the stimulation provision device 130. The analysis device can be, for example, a sequencer. The sequencer may be, for example, a next-generation sequencer or a sequencer that performs sequencing by the Sanger method.

The analysis system 10 can further include a two-dimensional mapping unit that performs two-dimensional mapping on the basis of a result of the association by the association unit by using a result of the analysis by the analysis device and the specimen image. The two-dimensional mapping unit may be provided as a component of the control unit 120 and can perform, for example, the two-dimensional mapping step described in the above section 1. The two-dimensional mapping unit can map, for example, information regarding the constituent of the cell on the specimen image. The information regarding the constituent of the cell may be, for example, the type or amount of the constituent of the cell. On the basis of the images obtained by the mapping, it is possible to grasp which cell at which position contains which molecule in what amount. That is, it is possible to combine the position information obtained by the imaging with quantitative information obtained by the sequence analysis, and thus it is possible to comprehensively analyze the molecules in the tissue sample while holding spatial information at the single-cell resolution.

The analysis system 10 may further include an output unit. The output unit can include, for example, a display device and/or a printing device. The control unit 120 can cause the output unit to output an image obtained by performing the analysis method according to the present technology, such as the specimen image acquired by the imaging element 110 and the mapping image generated by the two-dimensional mapping unit. Further, the control unit 120 can cause the output unit to output the result of the analysis by the analysis device (for example, the result of the sequencing process or the like).

### 3. Third embodiment (analysis surface)

The present technology also provides an analysis surface, in which a target capture molecule having a cleavable linker, a barcode sequence, and a target capture portion is immobilized to the analysis surface via the linker, and the barcode sequence is used to provide information regarding a position where the molecule having the barcode sequence is immobilized. An example of the analysis surface is the surface 101 of the analysis substrate 102 described in the above section 1, and the description of the surface is also applied to this embodiment.

Note that the present technology can also have the following configurations.
[1] An analysis method including:
   an imaging step of imaging a specimen in a state in which the specimen is being overlapped with a surface to which a molecule having a linker cleavable by stimulation, a barcode sequence, and a target capture portion is immobilized via the linker;
   an association step of associating a position of a cell with the barcode sequence of the molecule at the position by using a specimen image obtained by the imaging;
   a cleavage step of selectively stimulating the position of the cell to cleave the linker of the molecule at the position; and
   a binding step of binding the molecule released from the surface by the cleavage with a constituent of the cell via the target capture portion of the molecule.
[2] The analysis method according to [1], in which the specimen includes a tissue sample.
[3] The analysis method according to [1] or [2], in which in the cleavage step, the position of the cell is selectively stimulated so as not to cleave a linker of a molecule at a position other than the position of the cell.
[4] The analysis method according to any one of [1] to [3], in which the stimulation is light stimulation.
[5] The analysis method according to any one of [1] to [4], in which the binding step includes a moving step of moving the molecule toward the cell by applying an electric field, a magnetic field, or a centrifugal force.
[6] The analysis method according to any one of [1] to [4], in which the binding step includes a moving step of moving the molecule toward the cell by natural diffusion.
[7] The analysis method according to any one of [1] to [6], in which the binding step includes an incubation step of binding the molecule with the constituent of the cell.
[8] The analysis method according to any one of [1] to [7], further including an analysis step of analyzing a conjugate of the molecule and the constituent of the cell after the binding step.
[9] The analysis method according to [8], in which the conjugate is subjected to a sequencing process in the analysis step.
[10] The analysis method according to [8] or [9], in which the analysis step includes a two-dimensional mapping step of performing two-dimensional mapping on the basis of a result of the association in the association step by using a result of the analysis and the specimen image.
[11] An analysis system including:
   an analysis substrate having a surface to which a molecule having a linker cleavable by stimulation, a barcode sequence, and a target capture portion is immobilized via the linker;
   an imaging device that images a specimen overlapped with the analysis substrate;
   an association unit that associates a position of a cell selected in a specimen image obtained by the imaging with the barcode sequence of the molecule at the position; and
   a stimulation provision device that selectively stimulates the position of the cell, in which
   a conjugate in which the molecule released from the surface due to the stimulation binds with a constituent of the cell via the target capture portion of the molecule is used as an analysis target.
[12] The analysis system according to [11], in which the specimen includes a tissue sample.
[13] The analysis system according to [11] or [12], in which the stimulation provision device is configured to selectively stimulate the position of the cell, without cleaving a linker of a molecule at a position other than the position of the cell.
[14] The analysis system according to any one of [11] to [13], in which the stimulation provision device is a light irradiation device.
[15] The analysis system according to any one of [11] to [14], in which the analysis system includes an electric field application device, a magnetic field application device, or a centrifugal force application device that applies an electric field, a magnetic field, or a centrifugal force for moving, toward the cell, the molecule released from the surface due to the stimulation by the stimulation provision device.
[16] The analysis system according to any one of [11] to [15], further including an incubation device that prompts the molecule released from the surface due to the stimulation by the stimulation provision device to bind with the constituent of the cell.
[17] The analysis system according to any one of [11] to [16], further including an analysis device that analyzes the conjugate of the molecule released from the surface due to the stimulation by the stimulation provision device and the constituent of the cell.
[18] The analysis system according to [17], in which the analysis device is a sequencer.
[19] The analysis system according to [17] or [18], further including a two-dimensional mapping unit that performs two-dimensional mapping on the basis of a result of the association by the association unit by using a result of the analysis by the analysis device and the specimen image obtained by the imaging.
[20] An analysis surface, in which
   a molecule having a cleavable linker, a barcode sequence, and a target capture portion is immobilized to the analysis surface via the linker, and
   the barcode sequence is used to provide information regarding a position where the molecule having the barcode sequence is immobilized.

### REFERENCE SIGNS LIST

- 100: Molecule
- 101: Analysis surface
- 102: Substrate
- 103: Specimen
- 104: Substrate
- 110: Imaging element
- 120: Control unit
- 130: Stimulation provision device

## Claims

1. An analysis method comprising:
an imaging step of imaging a specimen in a state in which the specimen is being overlapped with a surface to which a molecule having a linker cleavable by stimulation, a barcode sequence, and a target capture portion is immobilized via the linker;
an association step of associating a position of a cell with the barcode sequence of the molecule at the position by using a specimen image obtained by the imaging;
a cleavage step of selectively stimulating the position of the cell to cleave the linker of the molecule at the position; and
a binding step of binding the molecule released from the surface by the cleavage with a constituent of the cell via the target capture portion of the molecule.

2. The analysis method according to claim 1, wherein the specimen includes a tissue sample.

3. The analysis method according to claim 1, wherein in the cleavage step, the position of the cell is selectively stimulated so as not to cleave a linker of a molecule at a position other than the position of the cell.

4. The analysis method according to claim 1, wherein the stimulation is light stimulation.

5. The analysis method according to claim 1, wherein the binding step includes a moving step of moving the molecule toward the cell by applying an electric field, a magnetic field, or a centrifugal force.

6. The analysis method according to claim 1, wherein the binding step includes a moving step of moving the molecule toward the cell by natural diffusion.

7. The analysis method according to claim 1, wherein the binding step includes an incubation step of binding the molecule with the constituent of the cell.

8. The analysis method according to claim 1, further comprising an analysis step of analyzing a conjugate of the molecule and the constituent of the cell after the binding step.

9. The analysis method according to claim 8, wherein the conjugate is subjected to a sequencing process in the analysis step.

10. The analysis method according to claim 8, wherein the analysis step includes a two-dimensional mapping step of performing two-dimensional mapping on a basis of a result of the association in the association step by using a result of the analysis and the specimen image.

11. An analysis system comprising:
an analysis substrate having a surface to which a molecule having a linker cleavable by stimulation, a barcode sequence, and a target capture portion is immobilized via the linker;
an imaging device that images a specimen overlapped with the analysis substrate;
an association unit that associates a position of a cell selected in a specimen image obtained by the imaging with the barcode sequence of the molecule at the position; and
a stimulation provision device that selectively stimulates the position of the cell, wherein
a conjugate in which the molecule released from the surface due to the stimulation binds with a constituent of the cell via the target capture portion of the molecule is used as an analysis target.

12. The analysis system according to claim 11, wherein the specimen includes a tissue sample.

13. The analysis system according to claim 11, wherein the stimulation provision device is configured to selectively stimulate the position of the cell, without cleaving a linker of a molecule at a position other than the position of the cell.

14. The analysis system according to claim 11, wherein the stimulation provision device is a light irradiation device.

15. The analysis system according to claim 11, wherein the analysis system includes an electric field application device, a magnetic field application device, or a centrifugal force application device that applies an electric field, a magnetic field, or a centrifugal force for moving, toward the cell, the molecule released from the surface due to the stimulation by the stimulation provision device.

16. The analysis system according to claim 11, further comprising an incubation device that prompts the molecule released from the surface due to the stimulation by the stimulation provision device to bind with the constituent of the cell.

17. The analysis system according to claim 11, further comprising an analysis device that analyzes the conjugate of the molecule released from the surface due to the stimulation by the stimulation provision device and the constituent of the cell.

18. The analysis system according to claim 17, wherein the analysis device is a sequencer.

19. The analysis system according to claim 17, further comprising a two-dimensional mapping unit that performs two-dimensional mapping on a basis of a result of the association by the association unit by using a result of the analysis by the analysis device and the specimen image obtained by the imaging.

20. An analysis surface, wherein
a molecule having a cleavable linker, a barcode sequence, and a target capture portion is immobilized to the analysis surface via the linker, and
the barcode sequence is used to provide information regarding a position where the molecule having the barcode sequence is immobilized.
